# EUROPEAN PATENT SPECIFICATION

(11) **EP 0 852 950 B1**
(45) Date of publication and mention of the grant of the patent: **23.07.2003**
(21) Application number: 98100213.2
(22) Date of filing: 08.01.1998
(51) Int. Cl.: A61K 35/12, A61K 35/36, A61P 31/00

(54) **Inflammatory tissue extract as nitrogen monoxide production suppressor**
Extrakt aus entzündetem Gewebe als Inhibitor der Stickstoffmonoxid-Produktion
Extrait de tissu inflammatoire comme inhibiteur de la production de monoxyde d'azote

(30) Priority: 08.01.1997 JP 1314697
(43) Date of publication of application: 15.07.1998
(73) Proprietor: Nippon Zoki Pharmaceutical Co., Ltd., Chuo-ku, Osaka (JP)
(72) Inventor: Ohno, Kousaku, c/o Nippon Zoki Pharm. Co., Ltd., Yashiro-cho, Katoh-gun, Hyogo (JP); Konishi, Jin-emon, c/o Nippon Zoki Pharm. Co. Ltd., Yashiro-cho, Katoh-gun, Hyogo (JP); Suehiro, Seishi, c/o Nippon Zoki Pharm. Co., Ltd., Yashiro-cho, Katoh-gun, Hyogo (JP)
(74) Representative: Hansen, Bernd, Dr. Dipl.-Chem.

(56) References cited:
- EP-A- 0 300 973
- EP-A- 0 315 591
- EP-A- 0 341 209
- PATENT ABSTRACTS OF JAPAN vol. 7, no. 114 (C-166), 18 May 1983 & JP 58 035117 A (NIHON ZOUKI SEIYAKU KK), 1 March 1983,
- PATENT ABSTRACTS OF JAPAN vol. 6, no. 158 (C-120), 19 August 1982 & JP 57 077697 A (NIPPON ZOKI PHARMACEUTICAL CO. LTD.), 15 May 1982,
- DATABASE WPI Week 7841 Derwent Publications Ltd., London, GB; AN 78-73614a XP002061660 & JP 53 101 515 A (NIPPON ZOKI PHARMACEUTICAL CO. LTD.) , 5 September 1978

## Description

### [Technical Field]

The present invention relates to a novel pharmacological action of an extract from inflammatory tissue inoculated with vaccinia virus. More particularly, it relates to a use for the preparation of a pharmaceutical agent such as a nitrogen monoxide production suppressor, the use for the preparation of a therapeutic agent for sepsis, use for a preparation as an antiendotoxinic agent and use for the preparation as a therapeutic agent for endotoxin shock containing the extract from inflammatory tissue inoculated with vaccinia virus as an effective component.

### [Prior Art]

It has been known that, against invasion from outside by virus, etc. and against progress of inner diseases state, living body produces various biofunction-regulating substances for maintaining its homeostasis and for regulating and normalizing the biofunctions by means of two phases consisting of a suppressing action to excessive reactions and an enhancing action to depression of functions. For example, there have been various reports on biofunction-regulating substances which are produced in inflammatory tissue inoculated with vaccinia virus, methods for extracting said substances from diseased tissues and pharmacological activities thereof (refer, for example, to the Japanese Examined Patent Publications Sho-63/039,572 B, Sho-63/025,600 B and Hei-03/043,279 B).

As to a drug which is actually available, there is a drug preparation of an extract from inflammatory rabbit skin inoculated with vaccinia virus. As mentioned in page 1,434 of "Drugs in Japan, Ethical Drugs" (published in August of 1994; edited by Japan Pharmaceutical Information Center; published by Yakugyo Jiho Co., Ltd.), this preparation is a drug containing non-protein active substances extracted and isolated from inflammatory tissues of rabbits inoculated with vaccinia virus, which has been allowed to be used against low back pain, neck-shoulder-arm syndromes, periarthritis scapulohumeralis, osteoarthritis, symptomatic neuralgia, itching accompanied with skin disorders (such as eczema, dermatitis and urticaria), allergic rhinitis, sequelae of subacute myelo-optico-neuropathy (such as coldness, pain and paresthesia/dysesthesia), etc., which is approved as an ethical drug in the forms of injections (subcutaneous, intramuscular and intravenous) and of tablets and commercially available.

As mentioned above, it has been known that an extract from inflammatory tissue inoculated with vaccinia virus has various pharmacological actions such as analgesic action, sedative action, antiallergic action and action of improving peripheral circulation. However, there has been no report at all concerning the novel pharmacological actions of the present invention such as a suppressing action to nitrogen monoxide production, a therapeutic action for sepsis, an antiendotoxinic action, a therapeutic action for endotoxin shock, etc.

### [Problems to be Solved by the Invention]

In sepsis and other serious bacterial infectious diseases, endotoxin (an intracellular toxin) is produced and, as a result of its action, shock symptom takes place. Endotoxin shows varieties of actions to living organism such as fever, leukocytosis, activation of complement and kinin system, induction of disseminated intravascular coagulation syndrome (DIC) and suppression of bone marrow. When the shock symptom by endotoxin progresses, an excessive hypotension, reduction of cardiac output, little urinary excretion, disturbance of consciousness, etc. are induced whereby the living body becomes a very serious state. It has been suggested that a rapid hypotension upon the endotoxin shock is due to an excessive nitrogen monoxide which is abnormally produced by the action of endotoxin with the vascular endothelial cells and there have been investigations on inhibitors for nitrogen monoxide synthesizing enzymes such as alginine derivatives with an object of achieving therapeutic agents for hypotension upon the endotoxin shock.

The present inventors have conducted various tests and studies on pharmacological activity of an extract from inflammatory tissue inoculated with vaccinia virus and, as a result, they have found that said extract has a suppressing action to death of cells and to an excessive production of nitrogen monoxide induced by endotoxin and also has an improving action to hypotension induced by endotoxin whereupon the present invention has been achieved.

### [Means to Solve the Problems]

An object of the present invention is to offer a novel use of a pharmaceutical composition containing an extract from inflammatory tissue inoculated with vaccinia virus such as the use for the preparation as a nitrogen monoxide production suppressor, a therapeutic agent for sepsis, an antiendotoxinic agent and a therapeutic agent for endotoxin shock.

### [Embodiments of the Invention]

The effective component of the pharmaceutical composition of the present invention is a non-protein biofunction-regulating substance extracted from inflammatory tissues inoculated with vaccinia virus. A drug preparation of an extract from inflammatory rabbit skin inoculated with vaccinia virus which is listed in the above-mentioned "Drugs in Japan, Ethical Drugs" has been approved as a pharmaceutical agent, put into the market and available in Japan. In addition, various extracts from inflammatory tissue inoculated with vaccinia virus mentioned in the references such as the above-mentioned patent publications can be utilized as the active substance of the present invention and their manufacturing methods and preferred doses are illustrated in the references as well.

With regard to the route of administration to the patient, subcutaneous, intramuscular and intravenous administrations by injection and oral administration by tablets are approved in the commercially available agent but it is also possible to administer the pharmaceutical dosage forms other than the above-mentioned ones which are optimum for the therapy depending upon the type of the disease. The dose is to be suitably decided depending upon the kind of an extract from inflammatory tissue inoculated with vaccinia virus while the dose which is approved in the commercially available preparation according to the above "Drugs in Japan, Ethical Drugs" (page 1,434) is, principally, 16 Neurotropin units per day and 3.6-7.2 Neurotropin units per day by oral administration and by injection, respectively. However, the dose may be appropriately increased or decreased depending upon the type of the disease, degree of seriousness, individual difference in the patients, method of administration, period of administration, etc.

As hereunder, results of the pharmacological tests concerning the novel pharmacological action of an extract from inflammatory tissue inoculated with vaccinia virus are given.

### (1) Cell Protective Action

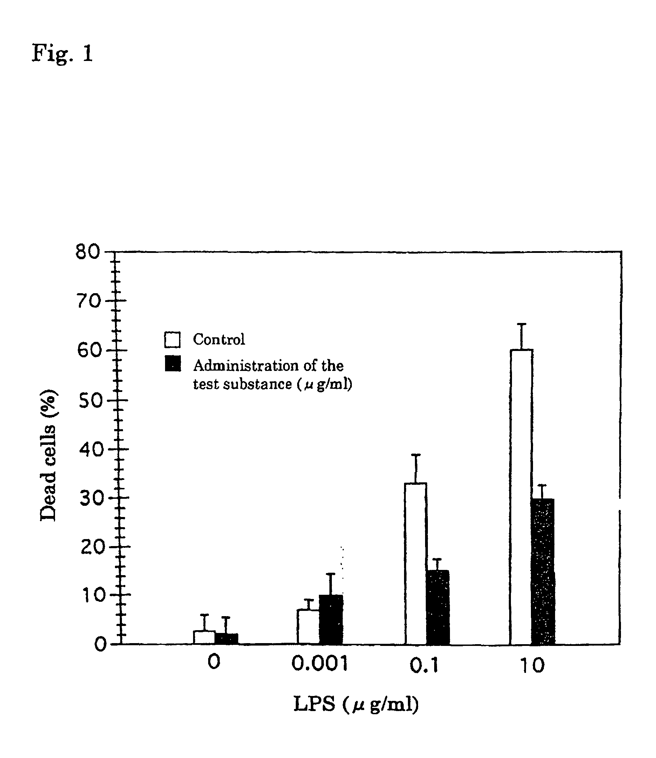

Endotoxin (lipopolysaccharide: LPS) in various concentrations was added to 2 x 10⁴ of human umbilical vein endothelial cells transplanted to a 3.5 cm incubating plate and said endothelial cells were seeded. Seven days after seeding, numbers of the cells were counted by a hemocytometer and a measurement was conducted whether the cells were alive or dead by a method dyeing with trypan blue. The substance of the present invention was made coexisted with this test system and the effect of the substance of the present on the death of endothelial cells by LPS was investigated. An example of the results thereof is shown in Fig. 1.

As shown in Fig. 1, LPS in a concentration of more than 0.1 µg/ml damaged the endothelial cells in a dose-dependent manner resulting in death of the cells. Against such LPS-induced cell death, the substance of the present invention prepared by a manufacturing method mentioned in Example 1 of the Japanese Examined Patent Publication Sho-63/039,572 B showed an excellent cell protective action whereupon the survival rate of the cells was significantly improved.

### (2) Inhibitory Action to Nitrogen Monoxide Production

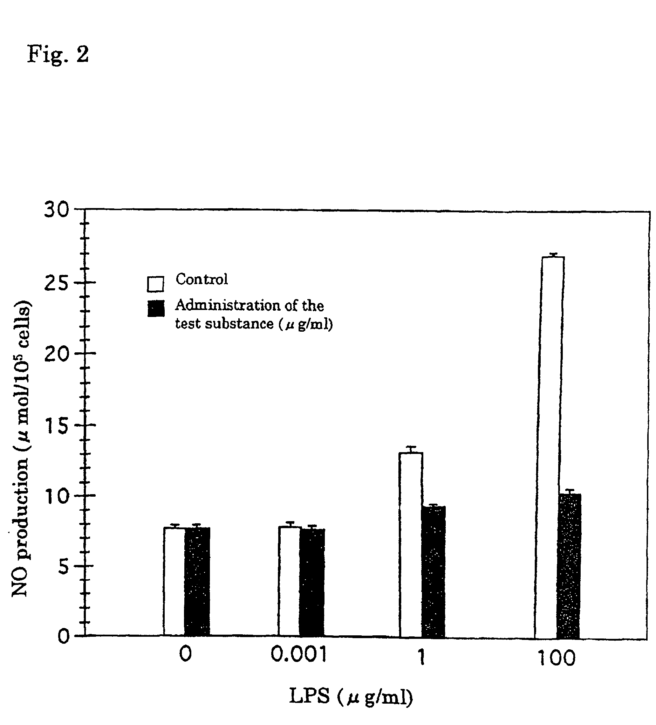

Human umbilical vein endothelial cells (2 x 10⁴ cells/well) were cultured on 96-well multi-plates in 200 ml of culture medium until the cells reached confluence. Then the medium was removed, a fresh medium containing LPS (0.001-100 mg/ml) and the above-mentioned substance of the present invention was added thereto and, after 24 hours, the amount of nitrogen monoxide produced thereby was measured by means of a color development analysis using a Griess reagent. Thus, 100 ml of the incubated cells were collected from each of the incubated wells, 100 ml of a Griess reagent (a 2% phosphoric acid solution containing 1% of sulfanilamide and 0.1% of N-1-naphthylethylenediamine dihydrochloride) was added thereto, the mixture was incubated at 25°C for ten minutes and the absorbance (optical density: 540 nm) was measured. Aqueous solution of sodium nitrite was used as a standard solution for investigating the inhibitory action of the substance of the present invention to the nitrogen monoxide production and an example thereof is shown in Fig. 2.

As one of the action mechanisms of the cell protective action of the substance of the present invention to the above-mentioned LPS-induced cell damage, an inhibitory action to nitrogen monoxide production by LPS was investigated. As shown in Fig. 2, production of nitrogen monoxide significantly increased in the presence of LPS at higher doses than 0.1 µg/ml and the substance of the present invention undoubtedly showed an inhibitory action to said nitrogen monoxide production induced by LPS. Further, the substance of the present invention (which was administered simultaneously with the administration of LPS) showed a suppressing action to an increase of NADPH-diaphorase-positive substance and an inducible nitrogen monoxide synthetase in lung, liver and kidney of LPS-treated mice. Accordingly, it was hereby suggested that the inhibitory action of the substance of the present invention to nitrogen monoxide production was due to inhibition of an expression of the inducible nitrogen monoxide synthetase.

### (3) Suppressing Action to LPS-Induced Lethal Toxicity

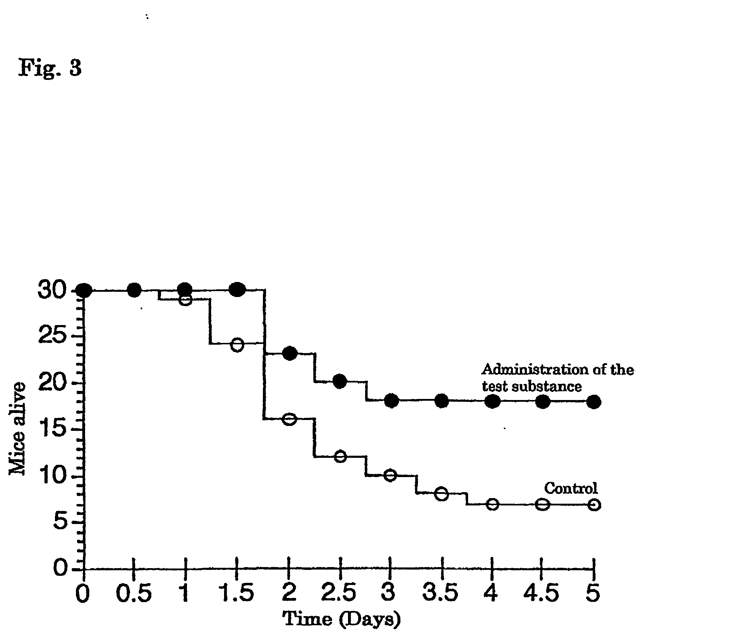

A solution of the substance of the present invention or a physiological saline solution buffered with phosphate was injected intraperitoneally to male ddy mice (25-30 g) together with LPS (0.75 mg/mouse). After the initial injection, a solution of the substance of the present invention (40 units/kg) or a phosphate-buffered physiological saline solution (control) was injected every 12 hours for five days. The mortality of mice were monitored twice a day for five days. An example of the result is shown in Fig. 3.

As shown in Fig. 3, survival rate of the mice injected with LPS decreased with a lapse of time while, in the group to which the substance of the present invention (a drug preparation of an extract from inflammatory rabbit skin inoculated with vaccinia virus; trade name: Neurotropin), a suppressing action to LPS-induced lethal toxicity was significantly noted. As such, like in the test of incubated endothelial cell line shown in Fig. 1, the suppressing action of the substance of the present invention to LPS-induced lethal toxicity was confirmed.

### (4) Improving Action to LPS-Induced Hypotension

Male Fischer rats (230-250 g) were anesthetized by an intraperitoneal injection of pentobarbital (40 mg/kg) and catheters were inserted into the artery for measurement of blood pressure and heart rate and also for administration of the substance of the present invention. After blood pressure and heart rate stablilized, the substance of the present invention (60 mg/kg) or a physiological saline solution (control) was injected intravenously. After 30 minutes, LPS (15 mg/kg) was injected and blood pressure was measured every ten minutes for three hours.

Average arterial blood pressure of the rats exposed to LPS decreased after about 40 minutes from the administration of LPS and, after 1.5-2.5 hours, a decrease of about 20% was noted. On the other hand, however, in the group to which a drug preparation of an extract from inflammatory rabbit skin inoculated with vaccinia virus was injected, no hypotension took place but a slight (around 5%) hypertension was observed.

### [Merit of the Invention]

It is clear from the above-mentioned results of the pharmacological tests that an extract from inflammatory tissue inoculated with vaccinia virus which is an effective component of the pharmaceutical composition of the present invention shows an excellent preventive action to the death of cells or animals induced by endotoxin (lipopolysaccharide: LPS) and also shows an evident inhibitory action to an excessive production of nitrogen monoxide induced by endotoxin. It has been suggested already that a sudden hypotension upon an endotoxin shock is due to an excessive production of nitrogen monoxide of the vascular endothelial cells induced by endotoxin and, in the above-mentioned pharmacological test in LPS-induced hypotension, the substance of the present invention having an inhibitory action to nitrogen monoxide production showed a preferred action of maintaining the normal blood pressure against hypotension after administration of LPS. In the above-mentioned pharmacological test (concerning a suppressing action to endotoxin-induced death, an inhibitory action to nitrogen monoxide production and an improving action to LPS-induced hypotension), an example of the test results is exemplified and such actions were also noted not only by the commercially available an extract from inflammatory rabbit skin inoculated with vaccinia virus but also by the extracts mentioned in the examples of the Japanese Examined Patent Publication (JP) Sho-63/039,572 B, by the extracts mentioned in the examples of JP Sho-63/025,600 B and by the extracts mentioned in the examples of JP Hei-03/043,279 B as well as by various extracts from inflammatory tissue inoculated with vaccinia virus.

In sepsis and other serious bacterial infectious diseases, endotoxin (an intracellular toxin) is produced and a shock symptom is induced by its action. Accordingly, the substance of the present invention having an excellent inhibitory action to the endotoxin-induced toxicity as mentioned above is quite useful for the treatment or the prevention of endotoxin-induced shock symptom, sepsis and various symptoms accompanied thereby. In addition, the substance of the present invention has an inhibitory action to abnormal nitrogen monoxide production during the diseased state and, therefore, it is also useful as a therapeutic and preventive agent to the diseases wherein an excessive nitrogen monoxide production is participated such as acute hypotension.

### [Examples]

An extract from inflammatory tissue e.g. skin tissue, in particular skin tissue of mammals inoculated with vaccinia virus which is effective component of the pharmaceutical composition of the present invention can be made into various pharmaceutical preparations by a suitable combination with various pharmaceutical carriers or diluents by conventional means whereby solid, semisolid, liquid or aerosol preparations for oral or parental use are obrained in the formulation, the substance of the present invention may be used solely or together with other pharmaceutically active components.

In the case of injections, it is possible to prepare the solutions or the suspensions in an aqueous and nonaqueous solvents such as distilled water for injection, physiological saline solution, Ringer's solution, plant oil, synthetic fatty acid glycerides, higher fatty acid esters, propylene glycol, etc.

In the case of preparations for oral administration, the substance of the present invention as it is or together with commonly-used excipients such as a suitable additive (e.g. lactose, mannitol, corn starch, potato starch, etc.) is mixed with binders such as crystalline cellulose, cellulose, gum arabicum, corn starch, gelatin, etc., disintegrating agents such as corn starch, potato starch, potassium carboxymethylcellulose, etc., lubricating agents such as talc, magnesium stearate, etc. and others including bulking agents, moisturizing agents, buffers, preservatives, perfumes and the like to give tablets, diluted powders, granules or capsules.

It is also possible, depending upon the type of the disease, to prepare the pharmaceutical preparations which are other than those which were mentioned already and are suitable for the therapy such as, for example, suppositories, inhalations, aerosol preparations, collyriums, ointments, poultices, etc.

### Brief Explanation of the Drawings:

Fig. 1 shows the inhibitory effect of the substance of the present invention to the death of endothelial cells caused by LPS.
Fig. 2 shows the inhibitory action of the substance of the present invention to an excessive production of nitrogen monoxide caused by LPS.
Fig. 3 shows the inhibitory action of the substance of the present invention to the lethal toxicity in mice caused by administration of LPS.

## Claims

1. Use of an extract from inflammatory tissue inoculated with vaccinia virus as an effective component for the preparation of a pharmaceutical composition suppressing the production of nitrogen monoxide induced by endotoxin.

2. Use of an extract from inflammatory tissue inoculated with vaccinia virus as an effective component for the preparation of a pharmaceutical composition effective as an antiendotoxinic agent.

3. Use of an extract from inflammatory tissue inoculated with vaccinia virus as an effective component for the preparation of a pharmaceutical composition for the treatment of sepsis.

4. Use of an extract from inflammatory tissue inoculated with vaccinia virus as an effective component for the preparation of a pharmaceutical composition for the treatment of endotoxin shock.

5. Use according to any of claims 1 to 4 wherein the inflammatory tissue is a skin tissue.

6. Use according to claim 5 wherein the inflammatory tissue is a skin tissue of mammals.

7. Use according to any of claims 1 to 6 wherein the pharmaceutical composition is formulated for injection

8. Use according to any of claims 1 to 6 wherein the pharmaceutical composition is formulated for oral administration.

## Patentansprüche

1. Verwendung eines Extrakts aus einem entzündeten Gewebe, das mit Vacciniavirus angeimpft wurde, als wirksame Komponente zur Herstellung einer pharmazeutischen Zusammensetzung zur Unterdrückung der Produktion von Stickstoffmonoxid, die durch Endotoxin induziert wird.

2. Verwendung eines Extrakts aus einem entzündeten Gewebe, das mit Vacciniavirus angeimpft wurde, als wirksame Komponente zur Herstellung einer pharmazeutischen Zusammensetzung die als Antiendotoxinmittel wirksam ist.

3. Verwendung eines Extrakts aus einem entzündeten Gewebe, das mit Vacciniavirus angeimpft wurde, als wirksame Komponente zur Herstellung einer pharmazeutischen Zusammensetzung zur Behandlung der Sepsis.

4. Verwendung eines Extrakts aus einem entzündeten Gewebe, das mit Vacciniavirus angeimpft wurde, als wirksame Komponente zur Herstellung einer pharmazeutischen Zusammensetzung zur Behandlung des Endotoxinschocks.

5. Verwendung gemäss einem der Ansprüche 1 bis 4, wobei das entzündete Gewebe ein Hautgewebe ist.

6. Verwendung gemäss Anspruch 5, wobei das entzündete Gewebe ein Hautgewebe von Säugern ist.

7. Verwendung gemäss einem der Ansprüche 1 bis 6, wobei die pharmazeutische Zusammensetzung zur Injektion formuliert ist.

8. Verwendung gemäss einem der Ansprüche 1 bis 6, wobei die pharmazeutische Zusammensetzung zur oralen Verabreichung formuliert ist.

## Revendications

1. Utilisation d'un extrait de tissu inflammatoire dans lequel a été inoculé le virus de la vaccine comme constituant actif pour la préparation d'une composition pharmaceutique supprimant la production de monoxyde d'azote induite par une endotoxine.

2. Utilisation d'un extrait de tissu inflammatoire dans lequel a été inoculé le virus de la vaccine comme constituant actif pour la préparation d'une composition pharmaceutique active comme agent anti-endotoxinique.

3. Utilisation d'un extrait de tissu inflammatoire dans lequel a été inoculé le virus de la vaccine comme constituant actif pour la préparation d'une composition pharmaceutique destinée au traitement de la septicémie.

4. Utilisation d'un extrait de tissu inflammatoire dans lequel a été inoculé le virus de la vaccine comme constituant actif pour la préparation d'une composition pharmaceutique destinée au traitement du choc endotoxinique.

5. Utilisation selon l'une quelconque des revendications 1 à 4, dans laquelle le tissu inflammatoire est un tissu cutané.

6. Utilisation selon la revendication 5, dans laquelle le tissu inflammatoire est un tissu cutané de mammifère.

7. Utilisation selon l'une quelconque des revendications 1 à 6, dans laquelle la composition pharmaceutique est formulée pour l'injection.

8. Utilisation selon l'une quelconque des revendications 1 à 6, dans laquelle la composition pharmaceutique est formulée pour l'administration orale.
